# EUROPEAN PATENT SPECIFICATION

(11) **EP 3 972 561 B1**
(45) Date of publication and mention of the grant of the patent: **24.07.2024**
(21) Application number: 20726241.1
(22) Date of filing: 28.04.2020
(51) Int. Cl.: A61K 9/00, A61K 9/06, A61K 45/06, A61K 47/06, A61K 47/44, A61K 31/727, A61K 31/568, A61P 17/00

(54) **PREPARATION FOR USE IN THE THERAPEUTIC TREATMENT OF ULCERATED SKIN LESIONS, WHEREIN THE PREPARATION IS ADMINISTERED TOPICALLY AND RELATIVE GROWTH FACTOR**
PRÄPARAT ZUR VERWENDUNG BEI DER THERAPEUTISCHEN BEHANDLUNG VON ULZERIERTEN HAUTLÄSIONEN ZUR TOPISCHEN VERABREICHUNG UND ZUGEHÖRIGER WACHSTUMSFAKTOR
PRÉPARATION DESTINÉE À ÊTRE UTILISÉE DANS LE TRAITEMENT THÉRAPEUTIQUE DE LÉSIONS CUTANÉES ULCÉREUSES, LA PRÉPARATION ÉTANT ADMINISTRÉE PAR VOIE TOPIQUE ET PAR UN FACTEUR DE CROISSANCE RELATIF

(30) Priority: 23.05.2019 IT 201900007187
(43) Date of publication of application: 30.03.2022
(73) Proprietor: Vignali, Massimo, 47900 Rimini (IT)
(72) Inventor: Vignali, Massimo, 47900 Rimini (IT)
(74) Representative: Busca, Andrea
(86) International application number: PCT/IB2020/053999
(87) International publication number: WO 2020/234672

(56) References cited:
- CN-A- 1 425 403
- US-A1- 2015 297 723

## Description

The present invention relates to a preparation for use in the therapeutic treatment of ulcerated skin lesions, wherein the preparation is administered topically and a relative growth factor. The invention is particularly useful for the treatment of the following:
- Trophic skin lesions due to pressure, due to stasis, due to decubitus.
- Skin lesions due to vascular ulcers.
- Skin lesions due to diabetic ulcers.
- Skin lesions due to abrasions, fissures, ulcers, wounds and sores.

### BACKGROUND

The ulcer is a continuous solution characterized by loss of substance which is secondary to necrotic processes of the skin and mucosae, with loss of the skin coating and underlying planes. It can be provoked by metabolic, vascular, lymphatic, trophic, infectious, traumatic and neoplastic causes. It is characterized by little or no tendency to the granulation and hence to repair process.

Currently there is no ideal resolutive medication for trophic skin ulcer and the relapse thereof. The medication should not adhere to the lesion or leave any residue on the bottom of the ulcer. The conventional treatment in the trophic ulcer of the lower limbs, according to the needs of the specific case, is of a medical-surgical type. The surgical treatment, made for the mechanical cleansing of the edges and of the bottom of the ulcer by means of scraping techniques with scalpels or curettes and gauzes, is useful to remove the necrotic tissue and the exudative component. The medical treatment is made with conventional therapies. The products used in this case are represented by: disinfectants, antimicrobials, foams, medicated gauzes, fibrinolytic agents, enzymes, collagen, fibrin glue, alginates, polyurethane, hydrocolloids, hydrogel, adhesive films, silver and sulfasalazine, hyaluronic acid, collagenase, fibrinolysin + deoxyribonucleases, growth factors, and many others. Other treatments are represented by the elastic compression of the lower limbs, the hyperbaric therapy, the laser therapy, the ultrasound therapy, and the phototherapy.

The following documents are known in patent literature.

GB1252377 describes a pharmaceutical composition for the treatment of skin lesions comprising salified heparinated polymyxin and cortisone steroid hormones.

It is observed that salified heparinated polymyxin is formed by two "conjugated" molecules, and therefore has the disadvantage of requiring a complex production process.

In addition, the cortisone steroid hormones block cell proliferation, and therefore are counterproductive in the treatment of ulcers.

Finally, this document teaches to combine female sex hormones (hydroxymethyltrimethyl aceto pregnadiene - example 4).

WO99/13717 describes the topical use of L-arginine, however it teaches to convey this active principle through liposomal structures for cellular absorption. However, this requires a complex production process and does not ensure a concentration with local action.

CN1425403 describes an ointment for the treatment of Leukoplakia which is a precancerous lesion. US 2015/297723 discloses a topical formulation for skin diseases which comprises testosterone propionate and heparin.

The skin or the mucosa in this case is intact, does not feature a loss of substance like in the ulcerated lesions. This document therefore does not provide any teaching on the treatment of ulcerated lesions, and on the contrary is aimed at avoiding promoting cell growth.

Therefore, the need for a drug for the resolutive treatment of ulcers and other skin lesions, which is easy to manufacture, low cost, practical to use and highly resolutive, remains latent in the sector.

The object of the present invention is therefore to meet this need.

A further object of the present invention is to provide a product for topical medication that meets one or more of the following requirements: keeping the ulcer hydrated and lubricated, relieving pain, stimulating the growth of granulation tissue, avoiding microbial growth and being cheap.

### SUMMARY

According to a first aspect, the invention relates to a preparation for use in the therapeutic treatment of ulcerated skin lesions, wherein the preparation is administered topically, characterized in that the preparation comprises at least unconjugated testosterone propionate, unconjugated sodium and/or calcium heparin, and unconjugated and non-conveyed L-arginine.

The form of the active ingredients used makes the preparation easily obtainable and widely available.

The applicant has discovered through experimentation that the hormone and the anticoagulant act in synergy with each other as an extremely effective growth factor, so much to heal the lesion in a short time.

The peculiarity of this mixture of elements is represented by a use other than the one usually indicated in pharmacology. For example, sodium or calcium heparin is a known anticoagulant, antithrombotic, antidislipidemic. On the other hand, in the composition of the present invention it has an angiogenetic action. The same applies to testosterone propionate, which unlike the cited literature is a male and non-female hormone, and whose activities stimulate cell growth.

The performance of the compound is further improved if it comprises unconjugated non-conveyed L-arginine.

In fact, in this form, arginine exhibits activities capable of increasing arteriolar vasodilation through the release of nitric oxide and stimulating cell growth locally through the release of active hormones; the same thing applies to vitamin components preferably present in the preparations which can and are considered, due to their action at the cellular level, prohormones.

The combined action of these active ingredients in the claimed form is not known in the traditional pharmacology, and indeed at first glance it is apparently not recommended in association with ulcerated lesions. In particular, the combined use of Heparin (an anticoagulant) and L-arginine (vasodilator effect) might seem counterproductive and not recommendable. Experimentation has shown that in association with the action of testosterone propionate there is a surprising response effect of the cell proliferative and vascular regenerative activity (angiogenesis) of the ulcerated skin tissues, insomuch that complete repair and/or closure and/or healing in the absence of scarring outcomes is obtained.

The invention therefore overcomes a prejudice in the sector.

The unconjugated form of the active ingredients enhances their synergy and the non-conveyed form of L-arginine allows the release of NITRIC OXIDE with a powerful arteriolar vasodilator function to be concentrated at the local cellular level and therefore enhanced. Therefore, the RELEASE of CELL GROWTH FACTORS such as for example GH:growth hormone takes place.

The tests showed that the preferred dosage is as follows: for 100 grams of preparation the dosage of unconjugated testosterone propionate is in the range 100-200 mg, and that of unconjugated sodium and/or calcium heparin is in the range 50,000 -500,000 I.U. in the total volume of 100gr. Where preferably the conversion in grams of the I.U.s is as follows: 1 I.U. = 0.01mg/ml (0.01 milligrams for each ml of preparation); therefore by indicating with Wmin the minimum weight and with Wmax the maximum weight of heparin in 100 grams of preparation and with V100 the volume of 100 gr. of preparation we obtain Wmin = 0.01*50.000*V100 and Wmax=0.01*500.000.

Preferably, moreover for 100 grams of preparation the dosage of unconjugated l-arginine is 0.5-3 gr.

According to some preferred embodiments, the preparation comprises at least the following three antibiotics: Chloramphenicol, Chlortetracycline, Neomycin. The pharmacological activity and the mechanism of these antibiotics ensures an action at the membrane and cytoplasmic prokaryotic level.

According to some preferred embodiments, the preparation comprises at least the following fat-soluble vitamins: Vitamin A (Retinol palmitate), Vit.D (ErgoCalciferol), Vit.E (d.l-Alpha-Tocopherol). They have the function of prohormones.

Preferably the preparation comprises at least the components and the relative dosages indicated in the following table, whereby, according to the invention, unconjugated non-conveyed L-arginine is present :

| | | | Quantity for 100 gr of preparation | |
|---|---|---|---|---|
| | | | Minimum | Maximum |
| GROWTH FACTORS | Hormone | Testosterone propionate | 100 mg | 200 mg |
| | Anticoagulant | Sodium and/or calcium heparin | 50,000 I.U. in the total volume of 100gr of preparation. | 500,000 I.U. in the total volume of 100gr. of preparation |
| ANTIMICROBIALS | Antibiotics | Chloramphenicol | 0.5 gr | 2 gr |
| | | Chlortetracycline | 0.5 gr | 2 gr |
| | | Neomycin | 0.5 gr | 2 gr |
| ANTIOXIDANTS | Fat-soluble vitamins | Vit.A | 500,000 I.U. | 1,000,000 I.U. |
| | | Vit.D | 100,000 I.U. | 200,000 I.U. |
| | | Vit.E | 100 mg | 200 mg |
| AUXILIARY COMPONENTS | Excipients | Lanolin | | |
| | | Paraffin | | |
| | | Vaseline | | |
| AMINO ACIDS (OPTIONAL) | | Unconjugated and non-conveyed L-Arginine | 0.5 gr | 3 gr |

The embodiments of more practical realisation and administration provide for the preparation to be in the form of an ointment. This therefore provides at least ointments, pastes, creams and gels, where ointments are preferred.

To make these forms, the preparation contains at least other necessary ingredients, such as excipients including, for example, but not limited to lanolin, paraffin, vaseline.

According to a second aspect thereof, the present invention relates to the use of at least unconjugated testosterone propionate and unconjugated sodium and/or calcium heparin, and optionally unconjugated and non-conveyed l-arginine, for the realisation of a preparation of the type indicated above.

According to a third aspect, the invention relates to a preparation given by the association in the unconjugated form between at least unconjugated testosterone propionate, unconjugated sodium and/or calcium heparin and unconjugated and non-conveyed l-arginine suitable for the release of cell growth factors.

### EXPERIMENTATION

The experimentation was carried out only through topical external use and in the following ways.
- Cleaning and disinfecting the lesion.
- Washing with physiological solution and drying.
- Applying (spreading) the preparation on the affected skin area.
- Flat medication with fat gauze and adsorbent silicone sponge.
- Closing the whole with sterile gauze by means of an occlusive bandage.
- Medicating and closing as above.

Application times:
- Apply the product evenly over the entire area affected by the lesion, once a day for the first seven days or on medical prescription.
- Continue the medication, after the first seven days, with an application of the product every two /three days or on medical prescription.
- Continue to medicate as above, until complete restitutio ad integrum of the affected area is obtained.
- The frequency of applications depends on the severity and tissue response times of the lesion.

### Manifestations:

In some cases, after application of the product, it was reported: itching and/or tingling.

### Contraindications:

At the moment none have been reported.

### Storage:

The preparation should preferably be stored below room temperature and protected from light.

The cases of skin ulcer of the lower limbs, treated with topical galenic preparations according to the invention being experimented, have all resulted in a positive outcome in a relatively short time. By way of demonstration, two clinical cases are described. The first clinical case is represented by a III degree skin pressure ulcer, located in the left foot, in the medial site, at the metatarsophalangeal joint of the first ray. The second clinical case documents the complete resolution of the III degree trophic skin ulcer of the right leg, located in the pretibial site.

### FIRST CASE:

95-year-old female patient suffering from skin lesion due to pressure on the left foot. The clinical picture is aggravated by various other factors highlighted by the following anamnesis:
Dementia, cardiomyopathy, bilateral carotid stenosis and chronic vascular cerebropathy, malnutrition and dehydration.

It was detected: cachectic appearance, significant sudden weight loss, sarcopenia, low-grade fever, sweating, arterial hypotension in clinostatism (BP 95/60), tachycardia (pulse rate 110/arrhythmic minute), lethargy, hyporeflexia, sunken eyes, conjunctival dryness, dryness of mucosae (xerostomia), non-toned and pale skin, contraction of diuresis/anuria (reported by the caregiver), oedema of upper limb and left lower limb, noises and thoracic crepitations, irregular respiratory rate, normal abdomen, venous access not available,

The patient was rehydrated with liquid infusion and fed with (hypodermic) micronutrient solutions via subcutaneous route, by means of the hypodermoclysis technique, for twenty days.

The III degree ulcer was treated by surgical toilet, local disinfectants and with topical applications of the preparation according to the present invention in the following formulation of ointment where the quantities are by weight for 100 gr. Of preparation:

| GROWTH FACTOR | Hormone | Testosterone | 100 mg |
|---|---|---|---|
| | Anticoagulant | Heparin | 50,000 I.U. |
| ANTIMICROBIALS | Antibiotics | Chloramphenicol | 1.5 gr |
| | | Chlortetracycline | 1.5 gr |
| | | Neomycin | 1.5 gr |
| ANTIOXIDANTS | Fat-soluble vitamins | Vit.A(Retinol palmitate) | 500,000 I.U. |
| | | Vit.D (ErgoCalciferol) | 100,000 I.U. |
| | | Vit.E(d.l-Alpha-Tocopherol) | 100 mg |
| AUXILIARY COMPONENTS | Excipients | Lanolin | Enough for 100 gr. |
| | | Liquid paraffin | |
| | | White vaseline | |
| AMINO ACIDS | | Arginine | 1 gr |

During the whole topical treatment, the patient never experienced any local nuisance or pain. Complete healing of the ulcer (restitutio ad integrum) was achieved with only seventeen topical applications of the product (in a relatively short time), in the absence of scarring outcomes, therefore with an excellent final aesthetic result.

During the whole period of local treatment, it was not detected and/or reported: side effects, allergic reactions, phlogosis and infections.

The granulation tissue appeared on the edges 48-72 hours after the first application.

The granulation tissue, from the first topical application onwards, responded immediately and continuously until the complete repair of the ulcer.

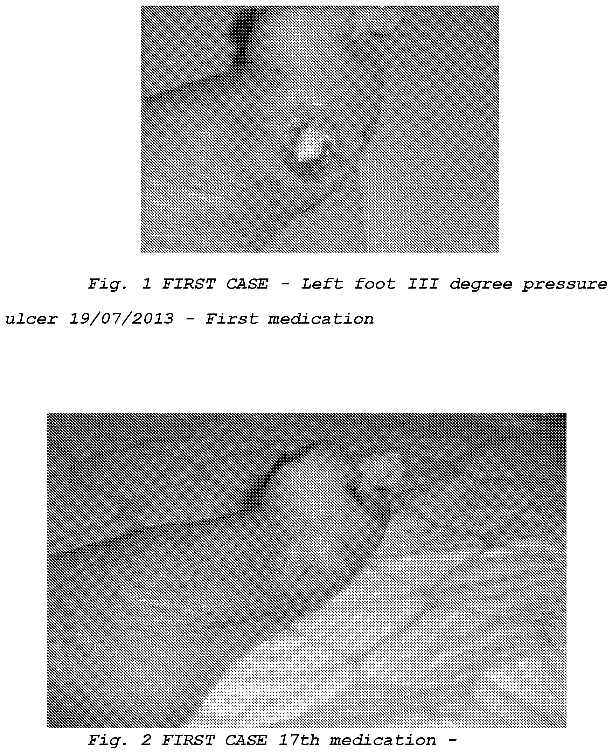

The photographic documentation of figures 1 and 2 testifies to the resolution of this first case.

The evolution was as follows:
- **20/07/2013:** Left foot ulcer was medicated;
   Appearance of the ulcer at the time of the initial check-up:
   1. Fibrinous tissue;
   2. Modest exudate;
   3. Absence of granulation tissue;
   4. Phlogosis of the margins and the skin around the lesion;
   5. Tendon exposure;
   6. Articular exposure with tendon fraying
   7. Dimensions: Width 4.5 cm, Length 4.5 cm
   8. III degree lesion
- **21/07/2013:** Medication of the left foot ulcer;
   Appearance of the lesion after one day of treatment, it is observed:
   improvement and reduction of skin phlogosis around the lesion, on the peripheral margins and on the bottom;
- **23/07/2013:** Medication of the left foot ulcer, it can be noted:
   improvement and reduction of skin phlogosis around the lesion, on the peripheral margins and on the bottom. Initial appearance of granulation tissue (patches) and increase in peripheral margins;
- **27/07/2013:** Medication of the left foot ulcer:
   Appearance of the lesion seven days after starting the treatment, it can be noted: marked improvement, initial appearance of the granulation tissue (patches) and increase in peripheral margins;
- **29/07/2013:** Medicated ulcer has a good trophism of the tissues with bed and granulating edges. Improved;
- **07/08/2013:** left foot pressure ulcer medicated.
- **09/08/2013:** Medication of the left foot ulcer it can be noted: marked improvement, reduction in size, presence of granulation tissue with relative growth of peripheral margins, surface re-epithelialisation and reduction of the ulcer.
- **20/08/2013:** Improved ulcer, presence of granulation tissue.
- **23/08/2013:** Medication of the left foot ulcer, resolution of the ulcer underway.
- **28/08/2013:** Medication of the left foot ulcer, it can be noted: marked improvement, continuous growth of peripheral margins and bottom, re-epithelialisation and reduction of the ulcer;
- **30/08/2013: *Left foot pressure ulcer with* re-epithelialisation *underway (closed).*** The patient feeds herself and hydrates herself orally sitting at the table. General improved conditions. Continue protein diet. BP 115/65, RATE 80 r, SAT.O2 97% in air.
- **19/09/2013: *Complete healing of the left foot pressure ulcer (restitutio* ad *integrum).*** The patient feeds herself and hydrates herself orally sitting at the table. General improved conditions (stable). Continuous protein diet BP 125/65, RATE 83 r., SAT.O2 94% in air.

### SECOND CASE:

66-year-old male patient suffering from a chronic skin ulcer on a venous vasculopathy basis, third degree, located in the anteromedial part of the lower third of the right leg.

The clinical picture is aggravated by various other factors highlighted by the following anamnesis:
The patient has been suffering for several years from type 2 diabetes mellitus, arterial hypertension, venous insufficiency in the lower limbs, obesity and glaucoma.

The trophic lesion appeared in November 2008 with progressive extension. The patient was included in the experimentation on February 11, 2009 after about three months of conventional, local and systemic medical therapy, without evident resolution. The ulcer was healed, with restitutio ad integrum in the absence of scarring outcomes, on June 05, 2009 after twenty-eight medications performed with the application of the preparation for topical use according to the present invention with the following formulation of ointment:

| GROWTH FACTOR | Hormone | Testosterone | 100 mg |
|---|---|---|---|
| | Anticoagulant | Heparin | 50,000 I.U. |
| ANTIMICROBIALS | Antibiotics | Chloramphenicol | 1.5 gr |
| | | Chlortetracycline | 1.5 gr |
| | | Neomycin | 1.5 gr |
| ANTIOXIDANTS | Fat-soluble vitamins | Vit.A(Retinol palmitate) | 500,000 I.U. |
| | | Vit.D (ErgoCalciferol) | 100,000 I.U. |
| | | Vit.E(d.l-Alpha-Tocopherol) | 100 mg |
| AUXILIARY COMPONENTS | Excipients | Lanolin | Enough for 100 gr. |
| | | Liquid paraffin | |
| | | White vaseline | |
| AMINO ACIDS | | Arginine | 1 gr |

### Recent pathological anamnesis

At the beginning of the experimentation, the patient had a chronic ulcer, on a venous vascular basis, located at the level of the medial anterior portion of the lower third of the right leg. The right leg shows evident subcutaneous oedema due to a likely hemolymphatic stasis caused by venous insufficiency of the lower limbs. This lesion, with the conventional medical treatments performed in the previous months, showed no improvement.

### Pharmacological anamnesis

At the beginning of the experimentation, the patient is undergoing a therapy with antihypertensive drugs, oral antidiabetic drugs, venotropic drugs, antilipemics, pump inhibitors, antiplatelet agents, and fans as needed.

### Tests performed

1. Blood tests: mild hyperglycaemia - urine pH8 and specific weight 1.014 - ESR 25 mm/hour, glycated hemoglobin 6.2%, glycosuria 0.07 gr / 24 h
2. Venous doppler ultrasound of lower limbs: (external test) - Venous insufficiency in the lower limbs.
3. Carotid doppler ultrasound: fibro-calcific plaques at the bulb-origin of the interior bilaterally, subcritical but markedly irregular on the left.
4. Cardiological examination: sinus bradycardia at 57 bpm. AV block. Incomplete RBBB. ECG abnormal. Unchanged from the previous ones. Stable heart detections. Routine checks of blood pressure. Therapy: Adalat 60, crono 1/day. Cardioaspirin, 1/day. Naprilene 20 mg, ½ cap. for 2 per day. Lasitone, 1/day. Torvast 10, 1/day. Neurontin, 3/day. Mepral 20, 1/day.
5. Vascular check-up: (external) Venous insufficiency of the lower limbs.
6. Endocrinological check-up: Type 2 diabetes. Therapy: Januvia.
7. Dietary check-up: appropriate diet
8. Eye check-up: Glaucoma :too 18-19 mmHg
9. Rheumatological check-up: multiple district arthrosis, lumbo-sacral discopathy
10. Periodic medical check-ups with his GP

### Physical Examination

***Site*** - The ulcer (Fig. 1) is located on the medial anterior cutaneous plane of the lower third of the right leg.
***Shape*** - Irregular.
***Dimension*** - Horizontal: 2 cm - Vertical: 2 cm - Depth: Loss of substance through the full thickness at the level of the skin coating.
***Number*-** Single.
***Margins*/*Edges*** - Irregular, Jagged, dropping.
***Lesion degree*** - III degree (Loss of substance through the full thickness in the skin coating).
***Inspection of the bottom*** - Upon inspection, the ulcerated lesion shows:
   1. Fibrinous tissue;
   2. Exudate;
   3. Absence of granulation tissue.
***Surrounding skin-*** The surrounding skin is inflamed and oedematous.
***Cultural examination*** - Not performed

### Previous treatment

Local - The patient reports previous conventional local medical treatments, for a period of about three months, with the following drugs:
1. Elase (Fibrinolysin + deoxyribonucleases)
2. Noruxol (Collagenase)
3. Connettivina ( Hyaluronic acid)

Systemic - The patient reports that in the previous months he has taken several systemic treatments with broad-spectrum antibiotics to treat a skin infection.

### Treatment applied during the experimentation

Local - The first medication was performed on February 11, 2009. After using hydrogen peroxide and/or povidone-iodine as a disinfectant, after using scalpels or curettes as mechanical cleansing and after using physiological solution or ringer solution as a washing and sterile gauzes as swabs, the preparation (Fig. 1) for topical use according to the invention was applied on the ulcer. Initially, the lesion was medicated every day for three to six days, then every three days for one to two weeks and finally every five days until its complete healing which took place on June 5, 2009. The preparation that is used promotes cleansing, the appearance of granulation tissue, neogenesis, maturation, remodelling and finally epithelialisation. The ulcer healed after twenty-eight applications in the absence of scarring outcomes. During the whole treatment period, no side effects, allergic reactions, phlogosis, infections were detected or reported. After just five days of treatment, granulation tissue (patches) with initial signs of reduction in the size of the ulcer appeared on the base, on the margins and on the edges of the lesion.

Systemic - The patient has never followed any type of systemic antibiotic therapy during the whole treatment period.

### COMMENTS

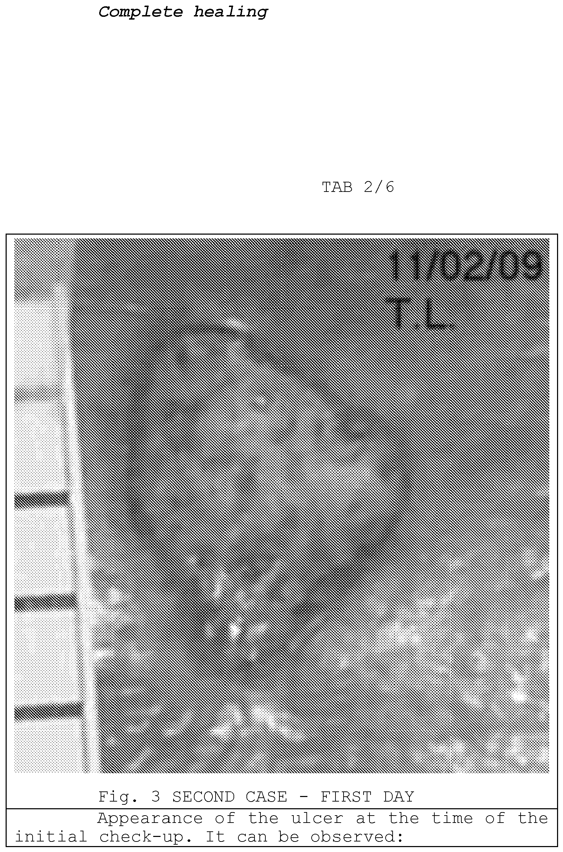

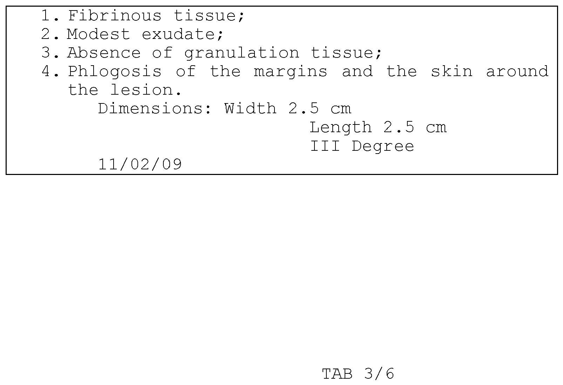

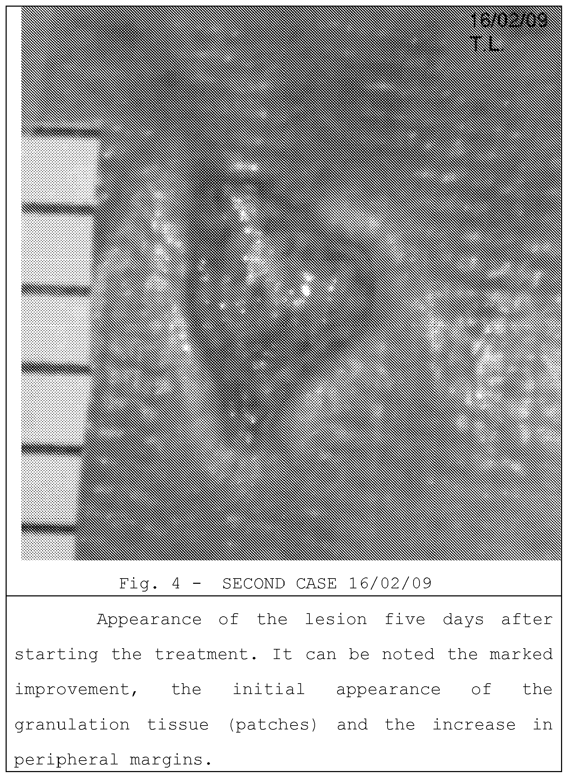

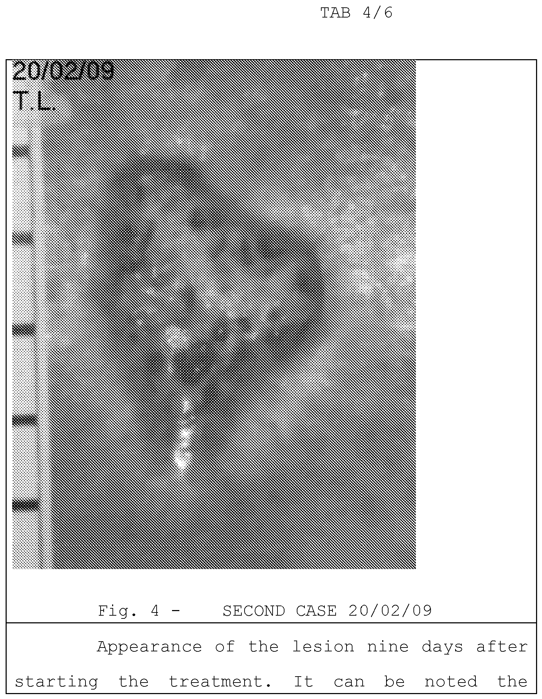

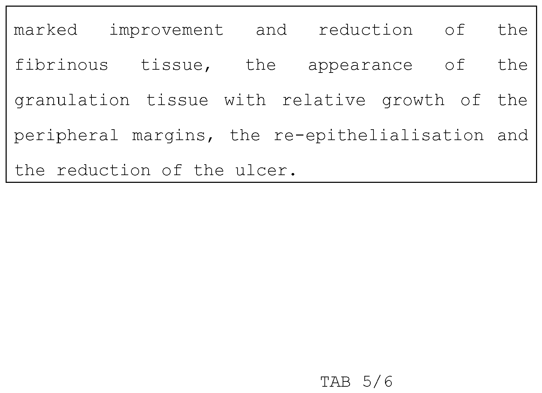

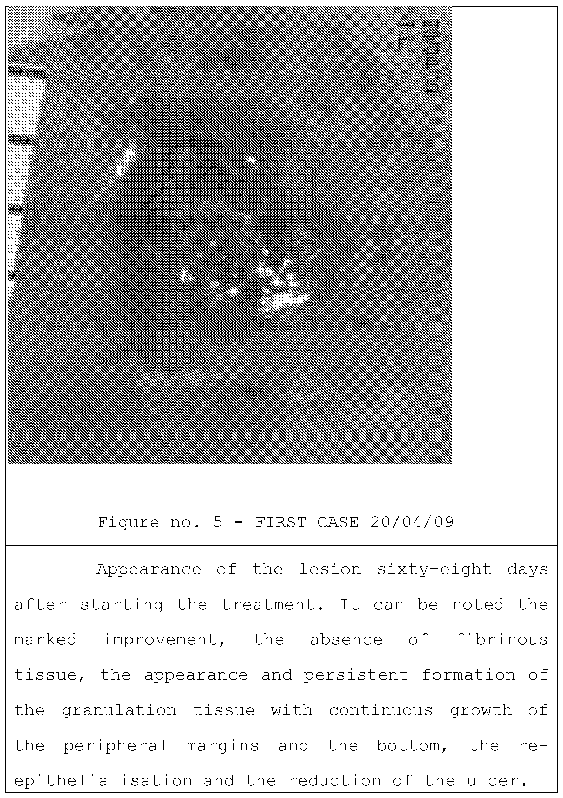

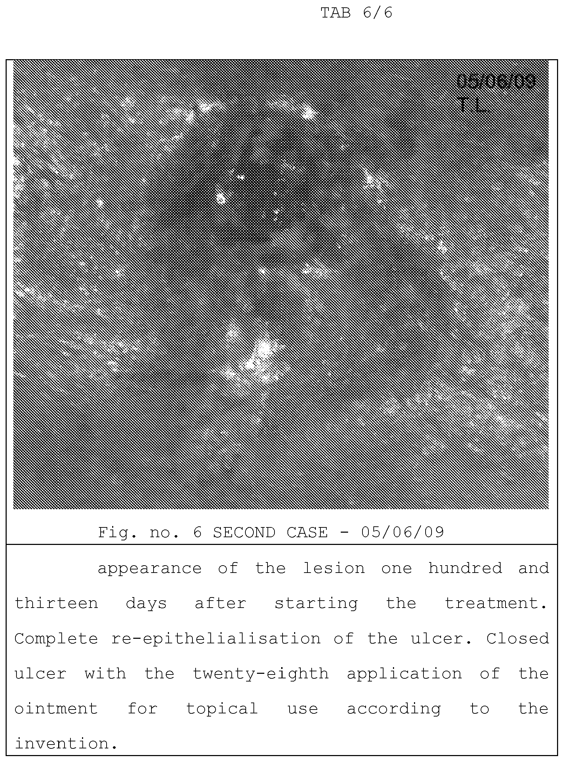

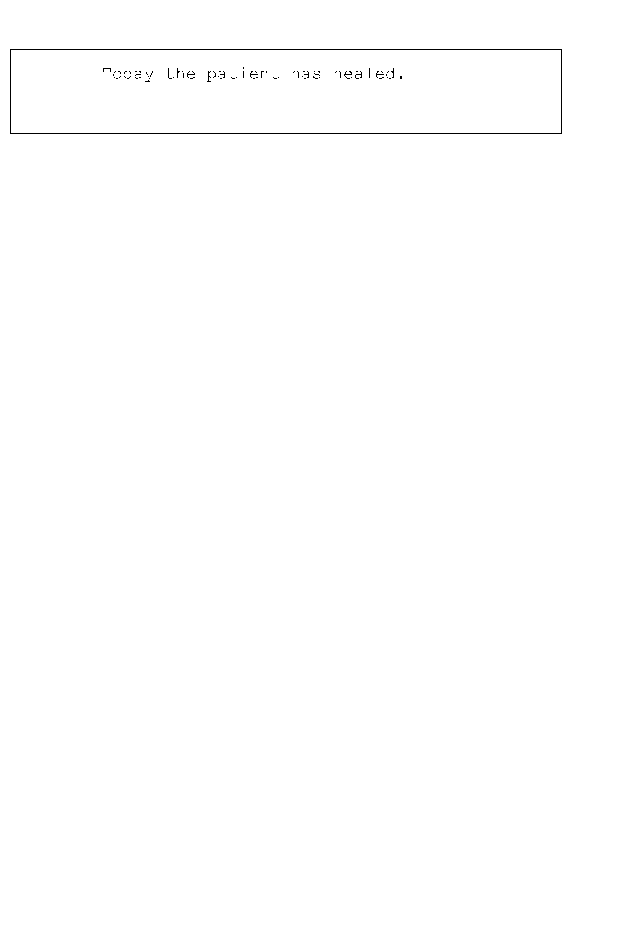

The product for topical use according to the present invention used to treat and heal the skin ulcer (Fig. 3-start, Fig. 4-end) has generated a significant advantage for the patient's well-being with considerable relief from the very first applications, in fact, he has no longer experienced neither local nuisance nor pain. The healing of the ulcer (Fig. 4) took place with restitutio ad integrum without scarring outcomes, therefore this new therapeutic approach, whose property is to ensure cleansing, granulation of the bottom and repair of the skin tissue, allowed to achieve excellent results with only twenty-eight medications, in a relatively short time. Based on the clinical experience of the inventor, made in recent years, it appears that the treatment according to the invention has a lower management cost and higher therapeutic results than the medical therapy currently implemented.

### RESULTS

The results obtained by applying the product for topical use are excellent. The lesion healed with twenty-eight applications without scarring outcomes and with a good aesthetic result. During the whole treatment period, no side effects, no allergic reactions, no phlogosis or no infections were detected and/or reported. The granulation tissue appeared after five days of treatment. The tissue, from the first topical application, responded immediately and continuously until the complete repair of the ulcer. The product promoted cleansing, the appearance of granulation tissue, neogenesis, maturation, remodeling and finally epithelialisation. The discomfort suffered by the patient, due to the pain and persistent nuisance, has significantly reduced until its complete resolution which was obtained after just a few days of local treatment.

The photographic documentation of figures 3 and 4 testifies to the resolution of this second case.

## Claims

1. A preparation for use in the therapeutic treatment of ulcerated skin lesions, wherein the preparation is administered topically, the preparation comprises at least unconjugated testosterone propionate, and unconjugated sodium and/or calcium heparin,
**characterized in that** it comprises unconjugated and non-conveyed L-arginine.

2. The preparation for use according to claim 1, **characterized in that** for 100 grams of preparation the dosage of unconjugated testosterone propionate is in the range 100-200 mg, and that of unconjugated sodium and/or calcium heparin is in the range 50,000-500,000 I.U. in the total volume of 100gr, where preferably the conversion in grams of the I.U.s is as follows: 1 I.U. = 0.01mg/ml (0.01 milligrams for each ml of preparation); therefore by indicating with Wmin the minimum weight and with Wmax the maximum weight of heparin in 100 grams of preparation and with V100 the volume of 100 gr. of preparation we obtain Wmin = 0.01*50.000*V100 and Wmax=0.01*500.000.

3. The preparation for use according to claim 2, **characterized in that** for 100 grams of preparation the dosage of unconjugated l-arginine is 0.5-3 gr.

4. The preparation for use according to any one of the preceding claims, **characterized in that** it comprises at least the following three antibiotics: Chloramphenicol, Chlortetracycline, Neomycin.

5. The preparation for use according to any one of the preceding claims, **characterized in that** it comprises at least the following fat-soluble vitamins: Vitamin A (e.g. Retinol palmitate), Vit.D (e.g. ErgoCalciferol) , Vit.E (d. 1-Alpha-Tocopherol).

6. The preparation for use according to the preceding claim, **characterized in that** it comprises at least the components and the relative dosages indicated in the following table:
| | | | Quantity for 100 gr of preparation | |
|---|---|---|---|---|
| | | | Minimum | Maximum |
| GROWTH FACTORS | Hormone | Testosterone propionate | 100 mg | 200 mg |
| | Anticoagulant | Sodium and/or calcium heparin | 50,000 I.U. in the total volume of 100gr of preparation. | 500,000 I.U. in the total volume of 100gr. of preparation |
| ANTIMICROBIALS | Antibiotics | Chloramphenicol | 0.5 gr | 2 gr |
| | | Chlortetracycline | 0.5 gr | 2 gr |
| | | Neomycin | 0.5 gr | 2 gr |
| ANTIOXIDANTS | Fat-soluble vitamins | VitA | 500,000 I.U. | 1,000,000 I.U. |
| | | Vit.D | 100,000 I.U. | 200,000 I.U. |
| | | ViLE | 100 mg | 200 mg |
| AUXILIARY COMPONENTS | Excipients | Lanolin | | |
| | | Paraffin | | |
| | | Vaseline | | |
| AMINO ACIDS (OPTIONAL) | | Unconjugated and non-conveyed L-Arginine | 0.5 gr | 3 gr |
where preferably the conversion in grams of the I.U.s is as follows: 1 I.U. = 0.01mg/ml (0.01 milligrams for each ml of preparation); therefore by indicating with Wmin the minimum weight and with Wmax the maximum weight of heparin in 100 grams of preparation and with V100 the volume of 100 gr. of preparation we obtain Wmin = 0.01*50.000*V100 and Wmax=0.01*500.000.

7. The preparation for use according to any one of the preceding claims, **characterized in that** it is an ointment.

8. A use of at least unconjugated testosterone propionate and unconjugated sodium and/or calcium heparin, and unconjugated and non-conveyed 1-arginine, for the realisation of a preparation for topical medication

9. A preparation given by the association in the unconjugated form between at least unconjugated testosterone propionate, unconjugated sodium and/or calcium heparin and unconjugated and non-conveyed 1-arginine which is suitable for the release of cell growth factors.

## Patentansprüche

1. Präparat zur Verwendung bei der therapeutischen Behandlung von ulzerierten Hautläsionen, wobei das Präparat topisch verabreicht wird und das Präparat mindestens unkonjugiertes Testosteronpropionat und unkonjugiertes Natrium- und/oder Calciumheparin umfasst, **dadurch gekennzeichnet, dass** es unkonjugiertes und nicht gefördertes L-Arginin enthält.

2. Präparat zur Verwendung nach Anspruch 1, **dadurch gekennzeichnet, dass** für 100 g des Präparats die Dosierung von unkonjugiertem Testosteronpropionat im Bereich von 100 bis 200 mg und die von unkonjugiertem Natrium- und/oder Calciumheparin im Bereich von 50.000 bis 500.000 I.E. im Gesamtvolumen von 100 g liegt, wobei vorzugsweise die Umrechnung in Gramm der I.E. wie folgt ist: 1 I.E. = 0,01 mg/ml (0,01 Milligramm für jeden ml der Zubereitung); wenn man also mit Wmin das Mindestgewicht und mit Wmax das Höchstgewicht von Heparin in 100 g der Zubereitung und mit V100 das Volumen von 100 g der Zubereitung angibt, erhält man Wmin 0,01*50.000*V100 und Wmax=0,01*500.000.

3. Zubereitung zur Verwendung nach Anspruch 2, **dadurch gekennzeichnet, dass** die Dosierung von unkonjugiertem L-Arginin 0,5 bis 3 g pro 100 g der Zubereitung beträgt.

4. Zubereitung zur Verwendung nach einem der vorstehenden Ansprüche, **dadurch gekennzeichnet, dass** sie mindestens die folgenden drei Antibiotika enthält: Chloramphenicol, Chlortetracyclin, Neomycin.

5. Zubereitung zur Verwendung nach einem der vorstehenden Ansprüche, **dadurch gekennzeichnet, dass** sie mindestens die folgenden fettlöslichen Vitamine enthält: Vitamin A (z. B. Retinolpalmitat), Vit. D (z. B. ErgoCalciferol), Vit. E (d. 1-Alpha-Tocopherol).

6. Zubereitung zur Verwendung gemäß dem vorstehenden Anspruch, **dadurch gekennzeichnet, dass** sie mindestens die in der folgenden Tabelle angegebenen Bestandteile und die entsprechenden Dosierungen umfasst:
| | | | Menge für 100 g der Zubereitung | |
|---|---|---|---|---|
| | | | Minimum | Maximum |
| WACHSTUMSFAKTOREN | Hormone | Testosteronpropi onat | 100 mg | 200 mg |
| | Antikoagulans | Natrium- und/oder Calciumheparin | 50.000 I.E. in der Gesamtmeng e von 100 g der Zubereitung. | 500.000 I.E. in der Gesamtmenge von 100 g der Zubereitung |
| ANTIMIKROBIELLE MITTEL | Antibiotika | Chloramphenicol | 0,5 g | 2 g |
| | | Chlortetracyclin | 0,5 g | 2 g |
| | | Neomycin | 0,5 g | 2 g |
| ANTIOXIDANTIEN | Fettlösliche Vitamine | Vit. A | 500.000 I.E. | 1.000.000 I.E. |
| | | Vit. D | 100.000 I.E. | 200.000 I.E. |
| | | Vit. E | 100 mg | 200 mg |
| HILFSKOMPONENTEN | Hilfsstoffe | Lanolin | | |
| | | Paraffin | | |
| | | Vaseline | | |
| AMINOSÄUREN (FAKULTATIV) | | Unkonjugiertes und nicht gefördertes L-Arginin | 0,5 g | 3 g |
wobei die Umrechnung in Gramm der I.E. vorzugsweise wie folgt erfolgt: 1 I.E. = 0,01 mg/ml (0,01 Milligramm für jeden ml der Zubereitung); wenn man also mit Wmin das Mindestgewicht und mit Wmax das Höchstgewicht von Heparin in 100 g der Zubereitung und mit V100 das Volumen von 100 g der Zubereitung angibt, erhält man Wmin 0,01*50,000*V100 und Wmax=0,01*500,000.

7. Zubereitung zur Verwendung nach einem der vorstehenden Ansprüche, **dadurch gekennzeichnet, dass** sie eine Salbe ist.

8. Verwendung von mindestens unkonjugiertem Testosteronpropionat und unkonjugiertem Natrium- und/oder Calciumheparin sowie unkonjugiertem und nicht gefördertem L-Arginin zur Herstellung eines Präparats für die topische Medikation

9. Präparat, das durch die Assoziation in der unkonjugierten Form zwischen mindestens unkonjugiertem Testosteronpropionat, unkonjugiertem Natrium- und/oder Calciumheparin und unkonjugiertem und nicht gefördertem L-Arginin gegeben ist, das für die Freisetzung von Zellwachstumsfaktoren geeignet ist.

## Revendications

1. Préparation destinée à être utilisée dans le traitement thérapeutique des lésions cutanées ulcérées, dans laquelle la préparation est administrée par voie topique, la préparation comprend au moins du propionate de testostérone non conjugué et de l'héparine sodique et/ou calcique non conjuguée,
**caractérisée en ce qu'**elle comprend de la L-arginine non conjuguée et non convoyée.

2. Préparation selon la revendication 1, **caractérisée en ce que** pour 100 grammes de préparation, le dosage du propionate de testostérone non conjugué est compris entre 100 et 200 mg, et celui de l'héparine sodique et/ou calcique non conjuguée est compris entre 50 000 et 500 000 U.I. dans le volume total de 100 g, où de préférence la conversion en grammes des U.I. se fait comme suit : 1 U.I. = 0,01mg/ml (0,01 milligramme pour chaque ml de préparation) ; par conséquent, en indiquant avec Wmin le poids minimal et avec Wmax le poids maximal de l'héparine dans 100 grammes de préparation et avec V100 le volume de 100 gr. de préparation, on obtient Wmin 0,01*50 000*V100 et Wmax=0,01*500 000.

3. Préparation destinée à être utilisée selon la revendication 2, **caractérisée en ce que** pour 100 grammes de préparation, le dosage de 1-arginine non conjuguée est de 0,5 à 3 grammes.

4. Préparation destinée à être utilisée selon l'une quelconque des revendications précédentes, **caractérisée en ce qu'**elle comprend au moins les trois antibiotiques suivants : Chloramphénicol, Chlortétracycline, Néomycine.

5. Préparation destinée à être utilisée selon l'une quelconque des revendications précédentes, **caractérisée en ce qu'**elle comprend au moins les vitamines liposolubles suivantes : Vitamine A (par ex. palmitate de rétinol), Vit.D (par ex. ErgoCalciférol), Vit.E (d. 1-Alpha-Tocophérol).

6. Préparation destinée à être utilisée selon la revendication précédente, **caractérisée en ce qu'**elle comprend au moins les composants et les dosages relatifs indiqués dans le tableau suivant :
| | | | Quantité pour 100 gr de préparation | |
|---|---|---|---|---|
| | | | Minimum | Maximum |
| FACTEURS DE CROISSANCE | Hormones | Propionate de testostérone | 100 mg | 200 mg |
| | Anticoagulant | Héparine sodique et/ou calcique | 50 000 U.I. dans le volume total de 100 gr de préparation. | 500 000 U.I. dans le volume total de 100 gr. de préparation |
| ANTIMICROBIENS | Antibiotiques | Chlorarnphénicol | 0,5 gr | 2 gr |
| | | Chlortétracyclin e | 0,5 gr | 2 gr |
| | | Néomycine | 0,5 gr | 2 gr |
| ANTIOXYDANTS | Vitamines liposolubles | Vit.A | 500 000 U.I. | 1 000 000 U.I. |
| | | Vit.D | 100 000 U.I. | 200 000 U.I. |
| | | Vit.E | 100 mg | 200 mg |
| COMPOSANTS AUXILIAIRES | Excipients | Lanoline | | |
| | | Paraffine | | |
| | | Vaseline | | |
| ACIDES AMINÉS (FACULTATIF) | | L-Arginine non conjuguée et non convoyée | 0,5 gr | 3 gr |
où, de préférence, la conversion en grammes des U.I. est la suivante : 1 U.I. = 0,01mg/ml (0,01 milligramme pour chaque ml de préparation) ; par conséquent, en indiquant avec Wmin le poids minimal et avec Wmax le poids maximal de l'héparine dans 100 grammes de préparation et avec V100 le volume de 100 gr. de préparation, on obtient Wmin 0,01*50 000*V100 et Wmax=0,01*500 000.

7. Préparation destinée à être utilisée selon l'une quelconque des revendications précédentes, **caractérisée en ce qu'**il s'agit d'une pommade.

8. Utilisation d'au moins du propionate de testostérone non conjugué et de l'héparine sodique et/ou calcique non conjuguée, et de la 1-arginine non conjuguée et non convoyée, pour la réalisation d'une préparation pour médicament topique.

9. Préparation obtenue par l'association, sous forme non conjuguée, d'au moins du propionate de testostérone non conjugué, de l'héparine sodique et/ou calcique non conjuguée et de la 1-arginine non conjuguée et non convoyée, qui convient à la libération de facteurs de croissance cellulaire.
